# EUROPEAN PATENT SPECIFICATION

(11) **EP 3 010 564 B1**
(45) Date of publication and mention of the grant of the patent: **14.10.2020**
(21) Application number: 14813602.1
(22) Date of filing: 23.06.2014
(51) Int. Cl.: A61M 1/12, A61F 2/24

(54) **ARTIFICIAL VENTRICLES**
KÜNSTLICHE VENTRIKEL
VENTRICULES ARTIFICIELS

(30) Priority: 21.06.2013 US 201313924472
(43) Date of publication of application: 27.04.2016
(73) Proprietor: Dalian Corvino Medical Technological Co. Ltd, Dalian 116019 (CN)
(72) Inventor: BIAN, Xiaoming, Dalian, Liaoning 116011 (CN); ZHENG, Frank, Kirkland, Washington 98034 (US)
(74) Representative: Hertin und Partner Rechts- und Patentanwälte PartG mbB
(86) International application number: PCT/CA2014/050598
(87) International publication number: WO 2014/201575

(56) References cited:
- WO-A1-00/59560
- WO-A2-2007/087014
- US-A- 3 768 931
- US-A- 3 768 931
- US-A- 5 332 403
- US-A- 5 332 403
- US-A1- 2009 292 160

## Description

### BACKGROUND OF THE INVENTION

### Field of the Invention

The present invention relates to ventricular assist devices and, in particular, to implantable artificial ventricles (eg. see US3768931).

### Description of the Related Art

It is known to use intra-aortic balloon pumps, operating in counterpulsation, to assist heart function. However, intra-aortic balloon pumps may be insufficient to sustain hemodynamics if the left heart is severely injured. There have accordingly been a number of alternative devices developed for assisting heart function in patients with chronic heart failure. For example, United States Patent Number 7,347,811 which issued on March 25, 2008 to Peters et al.. discloses a device for providing counter-pulsation heart assist by deforming the aorta. In a preferred embodiment, the deformation pressure is applied cyclically, preferably in synchrony with the diastolic period of the heart. The deformation pressure may be applied to the outer wall of the aorta or to a patch covering a resected opening in the wall of the aorta. There however remains a need for improved ventricular assist devices.

### SUMMARY OF THE INVENTION

It is an object of the present invention to provide an artificial ventricle and a method of artificial ventricle assist.

There is provided an artificial ventricle comprising an inlet for receiving blood, an outlet for discharging blood, and a chamber disposed between the inlet and the outlet. There is also a mechanism for actuating the artificial ventricle between an expanded configuration and a contracted configuration. In the expanded configuration blood flows into the inlet. In the contracted configuration blood flows out of the outlet. There may be a one-way valve at the outlet for preventing blood flow back into the chamber. The one-way valve may be a diaphragm valve. The chamber may have a resilient outer wall. The chamber may have an ovoid shape.

The mechanism for actuating the artificial ventricle between the expanded configuration and the contracted configuration may include a first pad disposed on the resilient outer wall of the chamber and a second pad disposed on the resilient outer wall of the chamber opposite of the first pad. The first pad may have a magnetic field generator and the second pad may have a material which is attracted to the magnetic field generator when the magnetic field generator generates a magnetic field. The second pad may move towards the first pad when the magnetic field generator generates a magnetic field, and thereby actuate the artificial ventricle to the contracted configuration by contracting the resilient outer wall of the chamber. The resilient outer wall of the chamber may actuate the artificial ventricle to the expanded configuration when the magnetic field generator is not generating a magnetic field and the material is not attracted to the magnetic field generator.

The mechanism for actuating the artificial ventricle between the expanded configuration and the contracted configuration may alternatively include a magnetic field generator integral with the resilient outer wall of the chamber and a material integral with the resilient outer wall of the chamber which is attracted to the magnetic field generator when the magnetic field generator generates a magnetic field. The material may move toward the magnetic field generator when the magnetic field generator generates a magnetic field and thereby actuate the artificial ventricle to the contracted configuration by contracting the resilient outer wall of the chamber. The resilient outer wall of the chamber may actuate the artificial ventricle to the expanded configuration when the magnetic field generator is not generating a magnetic field and the material is not attracted to the magnetic field generator.

The artificial ventricle may further include an electrical energy supply electrically connected to the magnetic field generator, a controller which drives the electric energy supply to either energize or de-energize the magnetic field generator, and an ECG signal generator which signals the controller when there is ventricular diastole and ventricular systole. The controller may drive the electrical energy supply to energize the magnetic field generator when the ECG signal generator signals the controller that there is ventricular diastole. The controller may drive the electrical energy supply to de-energize the magnetic field generator when the ECG signal generator signals the controller that there is ventricular systole.

There is also provided a method of left ventricle assist comprising removing a portion of the aorta distal of the native aortic valve and implanting an artificial ventricle to replace the removed portion of the aorta. The artificial ventricle comprises an inlet for receiving blood, an outlet for discharging blood, and a chamber disposed between the inlet and the outlet. There is also a mechanism for actuating the artificial ventricle between an expanded configuration and a contracted configuration. In the expanded configuration blood flows into the inlet. In the contracted configuration blood flows out of the outlet. The artificial ventricle is actuated to the expanded configuration during ventricular systole and the artificial ventricle is actuated to the contracted configuration during ventricular diastole.

There is further provided a method of right ventricle assist comprising removing a portion of the pulmonary artery distal of the native pulmonic valve and implanting an artificial ventricle to replace the removed portion of the pulmonary artery. The artificial ventricle comprises an inlet for receiving blood, an outlet for discharging blood, and a chamber disposed between the inlet and the outlet. There is also a mechanism for actuating the artificial ventricle between an expanded configuration and a contracted configuration. In the expanded configuration blood flows into the inlet. In the contracted configuration blood flows out of the outlet. The artificial ventricle is actuated to the expanded configuration during ventricular systole and the artificial ventricle is actuated to the contracted configuration during ventricular diastole.

### BRIEF DESCRIPTIONS OF DRAWINGS

The invention will be more readily understood from the following description of the embodiments thereof given, by way of example only, with reference to the accompanying drawings, in which:
Figure 1 is a fragmentary, sectional view of a biventricular failed heart provided with an implanted left artificial ventricle and an implanted right artificial ventricle;
Figure 2 is a perspective view of the left artificial ventricle of Figure 1;
Figure 3 is a sectional view of the left artificial ventricle of Figure 1 in an expanded configuration;
Figure 4 is a sectional view of the left artificial ventricle of Figure 1 in a contracted configuration;
Figure 5 is a flowchart showing a method for providing left ventricle assist;
Figure 6 is a flowchart showing a method for providing right ventricle assist;
Figure 7 is a flowchart showing operation of the artificial ventricles of Figure 1;
Figure 8 is a sectional view of another artificial ventricle in an expanded configuration;
Figure 9 is a sectional view of the artificial ventricle of Figure 8 in a contracted configuration;
Figure 10 is a sectional view of another artificial ventricle in an expanded configuration; and
Figure 11 is a sectional view of the artificial ventricle of Figure 10 in a contracted configuration.

### DESCRIPTIONS OF THE PREFERRED EMBODIMENTS

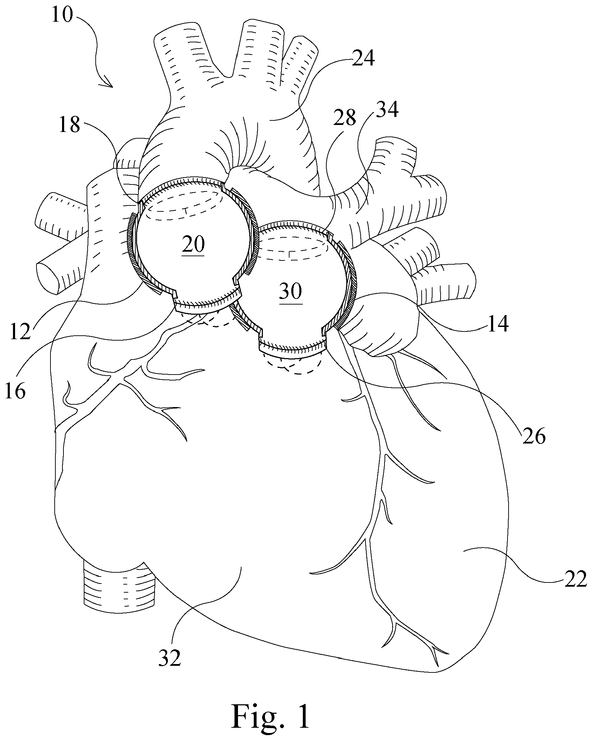

Referring to the drawings and first to Figure 1, there is shown a biventricular failed heart 10 provided with an implanted left artificial ventricle 12 and an implanted right artificial ventricle 14. The left artificial ventricle 12 generally comprises an inlet 16, an outlet 18, and a chamber 20 disposed between the inlet 16 and the outlet 18. The inlet 16 of the left artificial ventricle 12 is in fluid communication with a failed left ventricle 22 of the heart 10 and the outlet 18 of the left artificial ventricle is surgically connected to and in fluid communication with the aorta 24. The left artificial ventricle 12 accordingly assists blood flow from the left ventricle 22 to the aorta 24. More specifically, a portion of the aorta 24 distal of the native aortic valve (not shown) is removed and replaced by the left artificial ventricle 12. The inlet 16 of the left artificial ventricle 12 is connected to a proximal open end of the aorta 24. The outlet 18 of the left artificial ventricle 12 is connected to a distal open end of the aorta 24. The failed left ventricle 22 functions as a pathway for blood flow from the left atrium to the aorta 24 as well as a blood reservoir as will be described below.

Likewise the right artificial ventricle 14 generally comprises an inlet 26, an outlet 28, and a chamber 30 disposed between the inlet 26 and the outlet 28. The inlet 26 of the right artificial ventricle 14 is in fluid communication with a failed right ventricle 32 of the heart 10 and the outlet 28 of the left artificial ventricle is surgically connected to and in fluid communication with the pulmonary artery 34. The right artificial ventricle 14 accordingly allows for blood flow from the right ventricle 32 to the pulmonary artery 34. More specifically, a portion of the pulmonary artery 34 distal of the native pulmonic valve (not shown) is removed and replaced by the right artificial ventricle 14. The inlet 26 of the right artificial ventricle 14 is surgically connected to a proximal open end of the pulmonary artery 34. The outlet 28 of the right artificial ventricle 14 is connected to a distal open end of the pulmonary artery 34. The failed right ventricle 32 functions as a pathway for blood flow from the right atrium to the pulmonary artery 34 as well as a blood reservoir as will be described below.

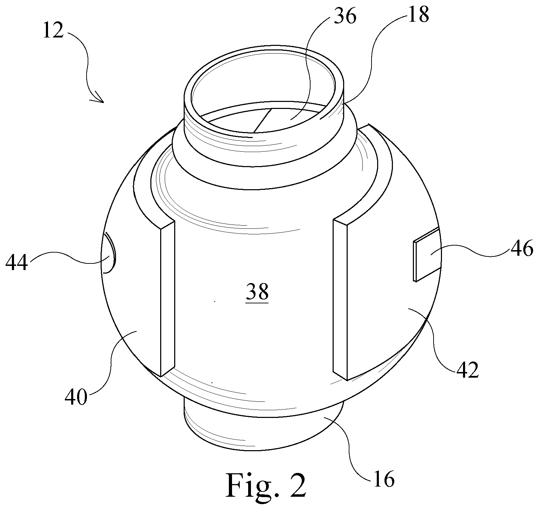

The left artificial ventricle 12 and the right artificial ventricle 14 have a substantially identical structure and function in a substantially identical manner. Accordingly, only the left artificial ventricle 12 is described in detail herein with the understanding that the right artificial ventricle 14 has a substantially identical structure and functions in a substantially identical manner. The left artificial ventricle 12 is shown in greater detail in Figure 2 and is provided with a one-way valve 36 at the outlet 18 thereof. The one-way valve 36 is a diaphragm check valve in this example but any suitable one-way valve may be used. There is a resilient outer wall 38 which defines the chamber 20 of the left artificial ventricle 12. The resilient outer wall 38 and therefore the left artificial ventricle 12 are substantially ovoid in this example as this shape may prevent thrombosis formation. However, in other examples, the left artificial ventricle 12 may have any suitable geometry.

There are flexible pads 40 and 42 on opposite sides of the resilient outer wall 38. In this example, the pads 40 and 42 are symmetrical in shape but one of the pads 40 is provided with a magnetic field generator in the form of an electromagnet 44 while the other one of the pads 42 is provided with a material 46 which will be attracted to the electromagnet 44 when the electromagnet 44 generates a magnetic field. The material 46 is a metal in this example. However, in other examples the material may be a magnetic field generator that generates a magnetic field having a polarity opposite to the magnetic field generated by the electromagnet 44. The electromagnet 44 and the material 46 may be disposed on or within their respective flexible pads 40 and 42. Alternatively, a magnetic field generator and a material which will be attracted to the magnetic field generator when the magnetic field generator generates a magnetic field may both be integral with the walls of the outer wall of the chamber.

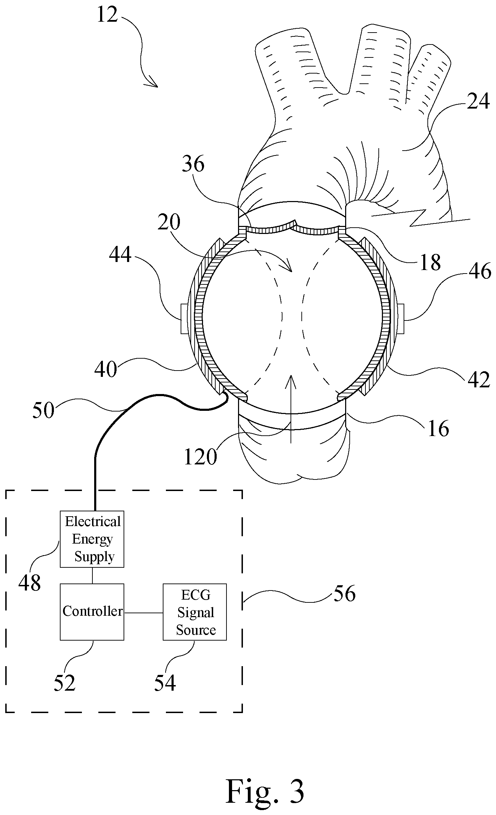

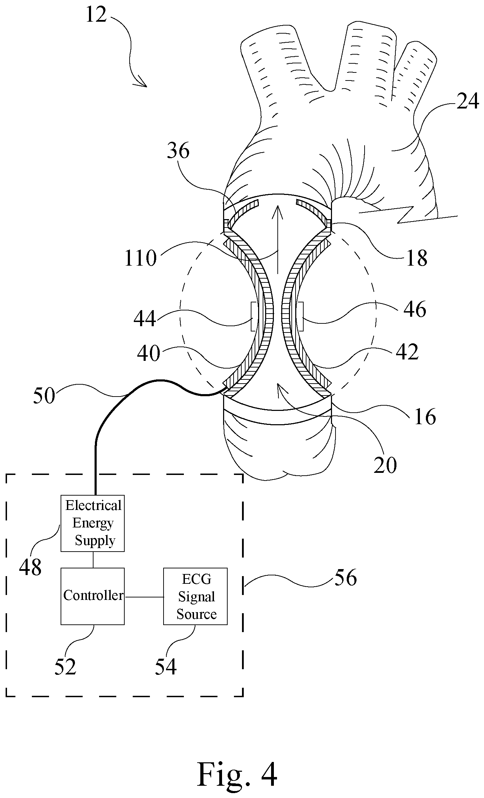

Referring now to Figures 3 and 4, operation of the left artificial ventricle 12 is shown. In Figure 3 the left artificial ventricle 12 is shown in an expanded configuration. In Figure 4 the left artificial ventricle 12 is shown in a contracted configuration. An electrical energy supply 48, for example a battery, is electrically connected with the electromagnet 44 via an electrical conductor which, in this example, is a wire 50. The electrical energy supply 48 is driven by a controller 52 which receives signals from an ECG signal source 54. The controller 52 drives the electrical energy supply 48 to energize the electromagnet 44 when the ECG signal source 54 signals that there is ventricular diastole and the controller 52 drives the electrical energy supply 48 to de-energize the electromagnet 44 when the ECG signal source 54 signals that there is ventricular systole. The electrical energy supply 48, the controller 52, and the ECG signal source 54 may all be part of an implanted pacemaker type device 56.

The left artificial ventricle 12 moves from the expanded configuration to the contracted configuration when the electromagnet 44 is energized. This is because the electromagnet 44 and the material 46 in the respective flexible pads 40 and 42 are then drawn towards one another. The pressure differential when the left artificial ventricle 12 is in the contracted configuration opens the one-way valve 36 and blood in the chamber 20 flows into the aorta 24 through the outlet 18 as indicated generally by arrow 110 in Figure 4. The left artificial ventricle 12 moves from the contracted configuration to the expanded configuration when the electromagnet 44 is de-energized. This is because the electromagnet 44 and the material 46 in the respective flexible pads 40 and 42 are no longer attracted to one another and the resilient nature of the resilient outer wall 38 actuates the left artificial ventricle 12 to move to the expanded configuration. The pressure differential when the left artificial ventricle 12 is in the expanded configuration closes the one-way valve 36. Accordingly, blood which flows into the chamber 20 through the inlet 16, as indicated generally by arrow 120 in Figure 3, is stored in the chamber and the chamber temporarily functions as a blood reservoir.

In operation, a portion of the ascending aorta is surgically removed and the left artificial ventricle 12 is implanted to replace the removed portion of the aorta. The two pads 40 and 42 are disposed on the resilient outer wall 38 of the chamber 20 on opposite sides of the resilient outer wall 38. One of the pads 40 is provided with the electromagnet 44 while the other one of the pads 42 is provided with the material 46 which is attracted to the electromagnet 44 when the electromagnet 44 generates a magnetic field. The electromagnet 44 is connected to the implanted pacemaker type device 56 through the wire 50. The implanted pacemaker type device 56 senses the patient's ECG and energizes and de-energizes the electromagnet 44 based on the patient's ECG. The electromagnet 44 is de-energized and the artificial ventricle 12 moves from the contracted configuration to the expanded configuration when the failed left ventricle 22 contracts as indicated by an R wave of the ECG. The one-way valve 36 at the outlet 18 simultaneously closes and the pressure inside the chamber 20 decreases while the aortic valve opens. The result is blood flow from the failed left ventricle 22 through the inlet 16 and into the chamber 20 of the left artificial ventricle 12. The electromagnet 44 is then energized and the artificial ventricle 12 moves from the expanded configuration to the contracted configuration when the failed left ventricle 22 relaxes as indicated by a T wave of the ECG. The one-way valve 36 at the outlet 18 simultaneously opens so the blood stored in the chamber 20 flows into the aorta 24. The left ventricle may accordingly function merely as a pathway for blood flowing from the left atrium to the left artificial ventricle 12 while the left artificial ventricle 12 functions as a pump.

The right artificial ventricle 14 functions in a substantially similar manner with the exception that a portion of the pulmonary trunk is surgically removed and the right artificial ventricle 14 is implanted to replace the removed portion of the pulmonary trunk. The right ventricle may then merely function as a pathway for blood flowing from the right atrium to the right artificial ventricle 14 while the right artificial ventricle 14 functions as a pump.

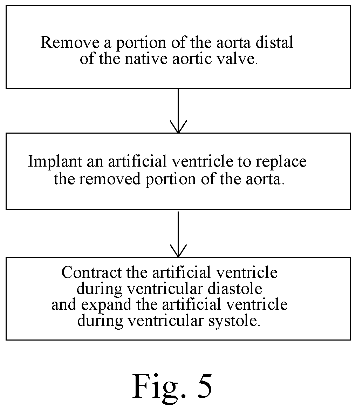

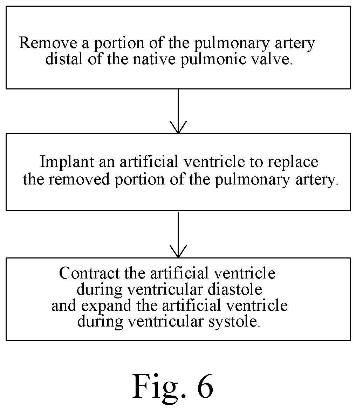

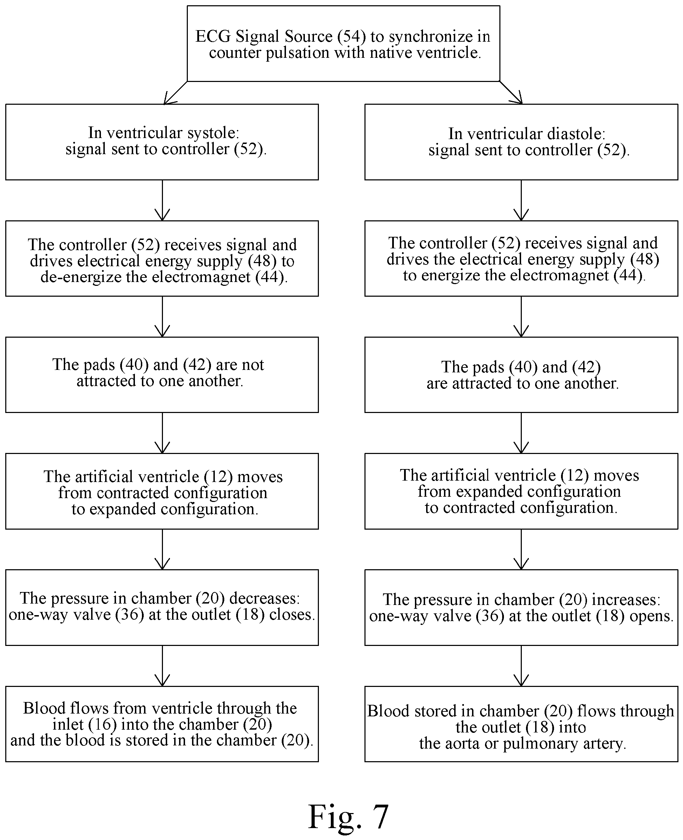

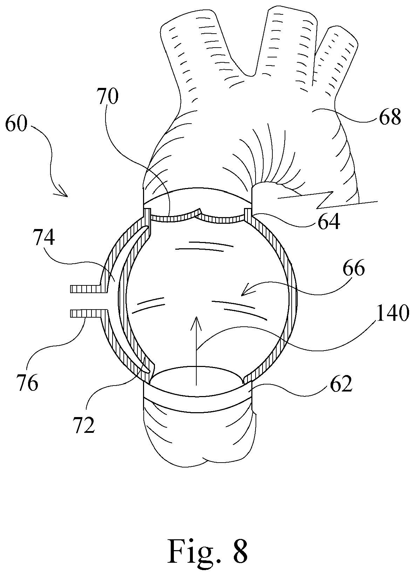

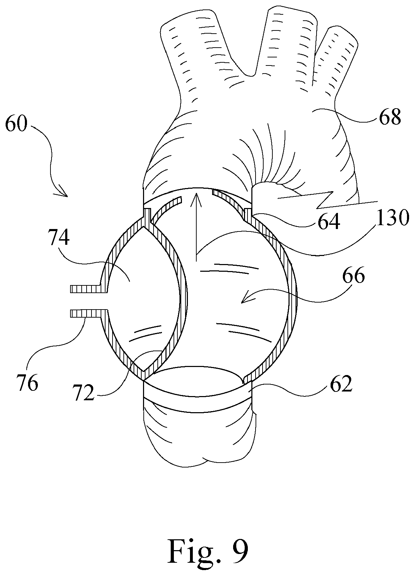

In the example shown in Figures 1 to 7, the mechanism for actuating the artificial ventricle between the expanded configuration and the contracted configuration is an electromagnetic mechanism. However, in other examples other mechanisms such as mechanical mechanisms, hydraulic mechanisms, electrical mechanisms, etc. may be used to actuate the artificial ventricle between the expanded configuration and the contracted configuration. For example, Figures 8 and 9 show another artificial ventricle 60 implanted in a failed left ventricle (not shown). The artificial ventricle 60 generally comprises an inlet 62, an outlet 64, and a main chamber 66 disposed between the inlet 62 and the outlet 64. The inlet 62 of the artificial ventricle 60 is in fluid communication with the failed left ventricle and the outlet 64 of the artificial ventricle 60 is in fluid communication with the aorta 68. There is a one-way valve 70 at the outlet 64 of the artificial ventricle 60. A resilient member 72 separates the main chamber 66 from an expansion chamber 74 which is in fluid communication with a fluid source via a conduit 76.

The artificial ventricle 60 may be actuated from an expanded configuration, shown in Figure 8, and a contracted configuration, shown in Figure 9, by introducing fluid into the expansion chamber 74. The artificial ventricle 60 may be actuated from the contracted configuration to the expanded configuration by withdrawing fluid from the expansion chamber 74. The pressure differential when the artificial ventricle 60 is in the contracted configuration opens the one-way valve 70 and blood in the main chamber 66 flows into the aorta 68 through the outlet 64 as indicated generally by arrow 130 in Figure 9. The pressure differential when the artificial ventricle 60 is in the expanded configuration closes the one-way valve 70. Accordingly, blood which flows into the chamber 20 through the inlet 62, as indicated generally by arrow 140 in Figure 8, is stored in the chamber and the chamber temporarily functions as a blood reservoir.

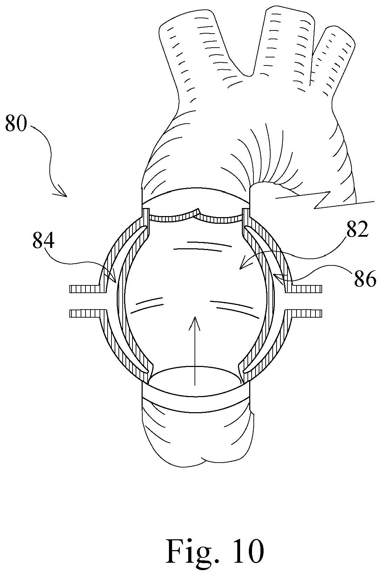

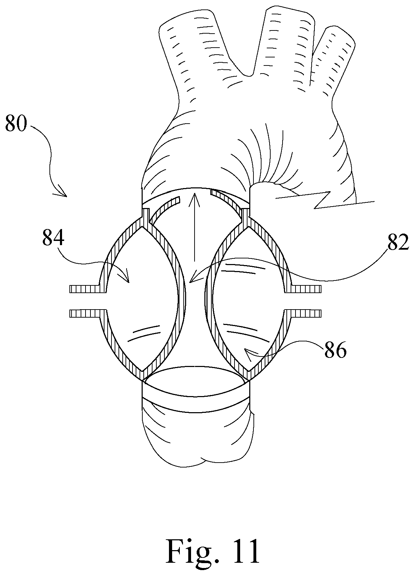

Figures 10 and 11 show yet another artificial ventricle 80 which is generally similar to the artificial ventricle 60 shown in Figures 8 and 9 with the exception that the main chamber 82 is disposed between expansion chambers 84 and 86.

The artificial ventricles disclosed herein may be fully implanted and replace the function of a late stage or fully failed ventricle. Blood volume per stroke may be designed into the shape and contraction means to achieve normal ventricle performance levels. Blood volume per stroke may be independent of ventricle performance. Providing the artificial ventricle with a one-way or back-flow prevention valve may improve efficiency by preventing blood flow back into the chamber.

It will be understood by a person skilled in the art that many of the details provided above are by way of example only, and are not intended to limit the scope of the invention which is to be determined with reference to the following claims.

## Claims

1. An artificial ventricle (12 or 14) comprising:
an inlet (16 or 26) for receiving blood;
an outlet (18 or 28) for discharging blood;
a chamber (20 or 30) disposed between the inlet (16 or 26) and the outlet (18 or 28);
a mechanism for actuating the artificial ventricle (12 or 14) between an expanded configuration and a contracted configuration, wherein in the expanded configuration blood flows into the inlet (16 or 26) and in the contracted configuration blood flows out of the outlet (18 or 28); and
**characterised in that** the chamber (20 or 30) has a resilient outer wall (38) and the mechanism for actuating the artificial ventricle (12 or 14) between the expanded configuration and the contracted configuration includes:
a first pad (40) disposed on the resilient outer wall (38) of the chamber (20 or 30), the first pad (40) having a magnetic field generator; and
a second pad (42) disposed on the resilient outer wall (38) of the chamber (20 or 30) opposite of the first pad (40), the second pad (42) having a material (46) which is attracted to the magnetic field generator when the magnetic field generator generates a magnetic field, wherein the second pad (42) moves towards the first pad (40) when the magnetic field generator generates a magnetic field and thereby actuates the artificial ventricle (12 or 14) to the contracted configuration by contracting the resilient outer wall (38) of the chamber (20 or 30), and wherein the resilient outer wall (38) of the chamber (20 or 30) actuates the artificial ventricle (12 or 14) to the expanded configuration when the magnetic field generator is not generating a magnetic field and the material (46) is not attracted to the magnetic field generator.

2. The artificial ventricle (12 or 14) as claimed in claim 1 comprising:
the inlet (16 or 26) for receiving blood;
the outlet (18 or 28) for discharging blood;
the chamber (20 or 30) disposed between the inlet (16 or 26) and the outlet (18 or 28);
the mechanism for actuating the artificial ventricle (12 or 14) between an expanded configuration and a contracted configuration, wherein in the expanded configuration blood flows into the inlet (16 or 26) and in the contracted configuration blood flows out of the outlet (18 or 28);
a one-way valve (36) at the outlet (18 or 28) for preventing blood flow back into the chamber (20 or 30), and
**characterised in that** the chamber (20 or 30) has the resilient outer wall (38) and the mechanism for actuating the artificial ventricle (12 or 14) between the expanded configuration and the contracted configuration includes:
the first pad (40) disposed on the resilient outer wall (38) of the chamber (20 or 30), the first pad (40) having the magnetic field generator; and
the second pad (42) disposed on the resilient outer wall (38) of the chamber (20 or 30) opposite of the first pad (40), the second pad (42) having the material (46) which is attracted to the magnetic field generator when the magnetic field generator generates a magnetic field, wherein the second pad (42) moves towards the first pad (40) when the magnetic field generator generates a magnetic field and thereby actuates the artificial ventricle (12 or 14) to the contracted configuration by contracting the resilient outer wall (38) of the chamber (20 or 30), and wherein the resilient outer wall (38) of the chamber (20 or 30) actuates the artificial ventricle (12 or 14) to the expanded configuration when the magnetic field generator is not generating a magnetic field and the material (46) is not attracted to the magnetic field generator.

3. The artificial ventricle (12 or 14) as claimed in claim 2 wherein the one-way valve (36) is a diaphragm valve.

4. The artificial ventricle (12 or 14) as claimed in claim 1 or 2 wherein the chamber (20 or 30) has an ovoid shape.

5. The artificial ventricle (12 or 14) as claimed in claim 1 or 2 further including:
an electrical energy supply (48) electrically connected to the magnetic field generator;
a controller (52) which drives the electrical energy supply (48) to either energize or de-energize the magnetic field generator; and
an ECG signal generator (54) which signals the controller (52) when there is ventricular diastole and ventricular systole, wherein the controller (52) drives the electrical energy supply (48) to energize the magnetic field generator when the ECG signal generator (54) signals the controller (52) that there is ventricular diastole and wherein the controller (52) drives the electrical energy supply (48) to de-energize the magnetic field generator when the ECG signal generator (54) signals the controller (52) that there is ventricular systole.

6. An artificial ventricle (12 or 14) comprising an inlet (16 or 26) for receiving blood;
an outlet (18 or 28) for discharging blood;
a chamber (20 or 30) disposed between the inlet (16 or 26) and the outlet (18 or 28);
a mechanism for actuating the artificial ventricle (12 or 14) between an expanded configuration and a contracted configuration, wherein in the expanded configuration blood flows into the inlet (16 or 26) and in the contracted configuration blood flows out of the outlet (18 or 28); and
**characterised in that** the chamber (20 or 30) has a resilient outer wall (38) and the mechanism for actuating the artificial ventricle (12 or 14) between the expanded configuration and the contracted configuration includes:
a magnetic field generator in the resilient outer wall (38) of the chamber (20 and 30); and
a material (46) in the resilient outer wall (38) of the chamber (20 or 30) which is attracted to the magnetic field generator when the magnetic field generator generates a magnetic field, wherein the material (46) moves towards the magnetic field generator when the magnetic field generator generates a magnetic field and thereby actuates the artificial ventricle (12 or 14) to the contracted configuration by contracting the resilient outer wall (38) of the chamber (20 or 30), and wherein the resilient outer wall (38) of the chamber (20 or 30) actuates the artificial ventricle (12 or 14) to the expanded configuration when the magnetic field generator is not generating a magnetic field and the material (46) is not attracted to the magnetic field generator.

7. The artificial ventricle (12 or 14) as claimed in claim 6 further including:
an electrical energy supply (48) electrically connected to the magnetic field generator;
a controller (52) which drives the electrical energy supply (48) to either energize or de-energize the magnetic field generator; and
an ECG signal generator (54) which signals the controller (52) when there is ventricular diastole and ventricular systole, wherein the controller (52) drives the electrical energy supply (48) to energize the magnetic field generator when the ECG signal generator (54) signals the controller (52) that there is ventricular diastole and wherein the controller (52) drives the electrical energy supply (48) to de-energize the magnetic field generator when the ECG signal generator (54) signals the controller (52) that there is ventricular systole.

## Patentansprüche

1. Künstliches Ventrikel (12 oder 14), umfassend:
einen Einlass (16 oder 26) zum Aufnehmen von Blut;
einen Auslass (18 oder 28) zum Abgeben von Blut;
eine Kammer (20 oder 30), die zwischen dem Einlass (16 oder 26) und dem Auslass (18 oder 28) angeordnet ist;
einen Mechanismus zum Betätigen des künstlichen Ventrikels (12 oder 14) zwischen einer ausgedehnten Konfiguration und einer zusammengezogenen Konfiguration, wobei in der ausgedehnten Konfiguration Blut in den Einlass (16 und 26) strömt und in der zusammengezogenen Konfiguration Blut aus dem Auslass (18 oder 28) strömt; und
**dadurch gekennzeichnet, dass** die Kammer (20 oder 30) eine elastische Außenwand (38) aufweist und der Mechanismus zum Betätigen des künstlichen Ventrikels (12 oder 14) zwischen der ausgedehnten Konfiguration und der zusammengezogenen Konfiguration Folgendes beinhaltet:
ein erstes Pad (40), das an der elastischen Außenwand (38) der Kammer (20 oder 30) angeordnet ist, wobei das erste Pad (40) einen Magnetfeldgenerator aufweist; und
ein zweites Pad (42), das an der elastischen Außenwand (38) der Kammer (20 oder 30) gegenüber dem ersten Pad (40) angeordnet ist, wobei das zweite Pad (42) ein Material (46) aufweist, das durch den Magnetfeldgenerator angezogen wird, wenn der Magnetfeldgenerator ein Magnetfeld erzeugt, wobei sich das zweite Pad (42) in Richtung des ersten Pads (40) bewegt, wenn der Magnetfeldgenerator ein Magnetfeld erzeugt und dadurch das künstliche Ventrikel (12 oder 14) in die zusammengezogene Konfiguration betätigt, indem er die elastische Außenwand (38) der Kammer (20 der 30) zusammenzieht, und wobei die elastische Außenwand (38) der Kammer (20 oder 30) das künstliche Ventrikel (12 oder 14) in die ausgedehnte Konfiguration betätigt, wenn der Magnetfeldgenerator kein Magnetfeld erzeugt und das Material (46) nicht durch den Magnetfeldgenerator angezogen wird.

2. Künstliches Ventrikel (12 oder 14) nach Anspruch 1, umfassend:
den Einlass (16 oder 26) zum Aufnehmen von Blut;
den Auslass (18 oder 28) zum Abgeben von Blut;
die Kammer (20 oder 30), die zwischen dem Einlass (16 oder 26) und dem Auslass (18 oder 28) angeordnet ist;
den Mechanismus zum Betätigen des künstlichen Ventrikels (12 oder 14) zwischen einer ausgedehnten Konfiguration und einer zusammengezogenen Konfiguration, wobei in der ausgedehnten Konfiguration Blut in den Einlass (16 und 26) strömt und in der zusammengezogenen Konfiguration Blut aus dem Auslass (18 oder 28) strömt;
ein Einwegeventil (36) an dem Auslass (18 oder 28) zum Verhindern eines Blutstroms zurück in die Kammer (20 oder 30) und
**dadurch gekennzeichnet, dass** die Kammer (20 oder 30) die elastische Außenwand (38) aufweist und der Mechanismus zum Betätigen des künstlichen Ventrikels (12 oder 14) zwischen der ausgedehnten Konfiguration und der zusammengezogenen Konfiguration Folgendes beinhaltet:
das erste Pad (40), das an der elastischen Außenwand (38) der Kammer (20 oder 30) angeordnet ist, wobei das erste Pad (40) den Magnetfeldgenerator aufweist; und
das zweite Pad (42), das an der elastischen Außenwand (38) der Kammer (20 oder 30) gegenüber dem ersten Pad (40) angeordnet ist, wobei das zweite Pad (42) das Material (46) aufweist, das durch den Magnetfeldgenerator angezogen wird, wenn der Magnetfeldgenerator ein Magnetfeld erzeugt, wobei sich das zweite Pad (42) in Richtung des ersten Pads (40) bewegt, wenn der Magnetfeldgenerator ein Magnetfeld erzeugt und dadurch das künstliche Ventrikel (12 oder 14) in die zusammengezogenen Konfiguration betätigt, indem er die elastische Außenwand (38) der Kammer (20 der 30) zusammenzieht, und wobei die elastische Außenwand (38) der Kammer (20 oder 30) das künstliche Ventrikel (12 oder 14) in die ausgedehnte Konfiguration betätigt, wenn der Magnetfeldgenerator kein Magnetfeld erzeugt und das Material (46) nicht durch den Magnetfeldgenerator angezogen wird.

3. Künstliches Ventrikel (12 oder 14) nach Anspruch 2, wobei das Einwegeventil (36) ein Membranventil ist.

4. Künstliches Ventrikel (12 oder 14) nach Anspruch 1 oder 2, wobei die Kammer (20 oder 30) eine Eiform aufweist.

5. Künstliches Ventrikel (12 oder 14) nach Anspruch 1 oder 2, ferner beinhaltend:
eine elektrische Energiezufuhr (48), die elektrisch mit dem Magnetfeldgenerator verbunden ist;
eine Steuerung (52), welche die elektrische Energiezufuhr (48) antreibt, um den Magnetfeldgenerator entweder zu erregen oder zu entregen; und
einen ECG-Signalgenerator (54), welcher der Steuerung (52) signalisiert, wenn eine ventrikuläre Diastole und eine ventrikuläre Systole vorhanden sind, wobei die Steuerung (52) die elektrische Energiezufuhr (48) antreibt, um den Magnetfeldgenerator zu erregen, wenn der ECG-Signalgenerator (54) der Steuerung (52) signalisiert, dass eine ventrikuläre Diastole vorhanden ist, und wobei die Steuerung (52) die elektrische Energiezufuhr (48) antreibt, um den Magnetfeldgenerator zu entregen, wenn der ECG-Signalgenerator (54) der Steuerung (52) signalisiert, dass eine ventrikuläre Systole vorhanden ist.

6. Elektrisches Ventrikel (12 oder 14), umfassend einen Einlass (16 oder 26) zum Aufnehmen von Blut;
einen Auslass (18 oder 28) zum Abgeben von Blut;
eine Kammer (20 oder 30), die zwischen dem Einlass (16 oder 26) und dem Auslass (18 oder 28) angeordnet ist;
einen Mechanismus zum Betätigen des künstlichen Ventrikels (12 oder 14) zwischen einer ausgedehnten Konfiguration und einer zusammengezogenen Konfiguration, wobei in der ausgedehnten Konfiguration Blut in den Einlass (16 und 26) strömt und in der zusammengezogenen Konfiguration Blut aus dem Auslass (18 oder 28) strömt; und
**dadurch gekennzeichnet, dass** die Kammer (20 oder 30) eine elastische Außenwand (38) aufweist und der Mechanismus zum Betätigen des künstlichen Ventrikels (12 oder 14) zwischen der ausgedehnten Konfiguration und der zusammengezogenen Konfiguration Folgendes beinhaltet:
einen Magnetfeldgenerator in der elastischen Außenwand (38) der Kammer (20 und 30); und
ein Material (46) an der elastischen Außenwand (38) der Kammer (20 oder 30), das durch den Magnetfeldgenerator angezogen wird, wenn der Magnetfeldgenerator ein Magnetfeld erzeugt, wobei sich das Material (46) in Richtung des Magnetfeldgenerators bewegt, wenn der Magnetfeldgenerator ein Magnetfeld erzeugt und dadurch das künstliche Ventrikel (12 oder 14) in die zusammengezogene Konfiguration betätigt, indem er die elastische Außenwand (38) der Kammer (20 der 30) zusammenzieht, und wobei die elastische Außenwand (38) der Kammer (20 oder 30) das künstliche Ventrikel (12 oder 14) in die ausgedehnte Konfiguration betätigt, wenn der Magnetfeldgenerator kein Magnetfeld erzeugt und das Material (46) nicht durch den Magnetfeldgenerator angezogen wird.

7. Künstliches Ventrikel (12 oder 14) nach Anspruch 6, ferner beinhaltend:
eine elektrische Energiezufuhr (48), die elektrisch mit dem Magnetfeldgenerator verbunden ist;
eine Steuerung (52), welche die elektrische Energiezufuhr (48) antreibt, um den Magnetfeldgenerator entweder zu erregen oder zu entregen; und
einen ECG-Signalgenerator (54), welcher der Steuerung (52) signalisiert, wenn eine ventrikuläre Diastole und eine ventrikuläre Systole vorhanden sind, wobei die Steuerung (52) die elektrische Energiezufuhr (48) antreibt, um den Magnetfeldgenerator zu erregen, wenn der ECG-Signalgenerator (54) der Steuerung (52) signalisiert, dass eine ventrikuläre Diastole vorhanden ist, und wobei die Steuerung (52) die elektrische Energiezufuhr (48) antreibt, um den Magnetfeldgenerator zu entregen, wenn der ECG-Signalgenerator (54) der Steuerung (52) signalisiert, dass eine ventrikuläre Systole vorhanden ist.

## Revendications

1. Ventricule artificiel (12 ou 14) comprenant :
une entrée (16 ou 26) pour recevoir le sang ;
une sortie (18 ou 28) pour évacuer le sang ;
une chambre (20 ou 30) disposée entre l'entrée (16 ou 26) et la sortie (18 ou 28) ;
un mécanisme pour actionner le ventricule artificiel (12 ou 14) entre une configuration étendue et une configuration contractée, dans lequel, dans la configuration étendue, le sang s'écoule dans l'entrée (16 ou 26) et, dans la configuration contractée, le sang s'écoule hors de la sortie (18 ou 28) ; et
**caractérisé en ce que** la chambre (20 ou 30) a une paroi externe élastique (38) et le mécanisme pour actionner le ventricule artificiel (12 ou 14) entre la configuration étendue et la configuration contractée comporte :
un premier tampon (40) disposé sur la paroi externe élastique (38) de la chambre (20 ou 30), le premier tampon (40) ayant un générateur de champ magnétique ; et
un second tampon (42) disposé sur la paroi externe élastique (38) de la chambre (20 ou 30) opposé au premier tampon (40), le second tampon (42) ayant un matériau (46) qui est attiré vers le générateur de champ magnétique lorsque le générateur de champ magnétique génère un champ magnétique, dans lequel le second tampon (42) se déplace vers le premier tampon (40) lorsque le générateur de champ magnétique génère un champ magnétique et actionne ainsi le ventricule artificiel (12 ou 14) vers la configuration contractée en contractant la paroi externe élastique (38) de la chambre (20 ou 30), et dans lequel la paroi externe élastique (38) de la chambre (20 ou 30) actionne le ventricule artificiel (12 ou 14) vers la configuration étendue lorsque le générateur de champ magnétique ne génère pas de champ magnétique et que le matériau (46) n'est pas attiré vers le générateur de champ magnétique.

2. Ventricule artificiel (12 ou 14) selon la revendication 1, comprenant :
l'entrée (16 ou 26) pour recevoir le sang ;
la sortie (18 ou 28) pour évacuer le sang ;
la chambre (20 ou 30) disposée entre l'entrée (16 ou 26) et la sortie (18 ou 28) ;
le mécanisme pour actionner le ventricule artificiel (12 ou 14) entre une configuration étendue et une configuration contractée, dans lequel, dans la configuration étendue, le sang s'écoule dans l'entrée (16 ou 26) et, dans la configuration contractée, le sang s'écoule hors de la sortie (18 ou 28) ;
une soupape unidirectionnelle (36) à la sortie (18 ou 28) pour empêcher un reflux de sang dans la chambre (20 ou 30), et
**caractérisé en ce que** la chambre (20 ou 30) a la paroi externe élastique (38) et le mécanisme pour actionner le ventricule artificiel (12 ou 14) entre la configuration étendue et la configuration contractée comporte :
le premier tampon (40) disposé sur la paroi externe élastique (38) de la chambre (20 ou 30), le premier tampon (40) ayant le générateur de champ magnétique ; et
le second tampon (42) disposé sur la paroi externe élastique (38) de la chambre (20 ou 30) opposé au premier tampon (40), le second tampon (42) ayant le matériau (46) qui est attiré vers le générateur de champ magnétique lorsque le générateur de champ magnétique génère un champ magnétique, dans lequel le second tampon (42) se déplace vers le premier tampon (40) lorsque le générateur de champ magnétique génère un champ magnétique et actionne ainsi le ventricule artificiel (12 ou 14) vers la configuration contractée en contractant la paroi externe élastique (38) de la chambre (20 ou 30), et dans lequel la paroi externe élastique (38) de la chambre (20 ou 30) actionne le ventricule artificiel (12 ou 14) vers la configuration étendue lorsque le générateur de champ magnétique ne génère pas de champ magnétique et que le matériau (46) n'est pas attiré vers le générateur de champ magnétique.

3. Ventricule artificiel (12 ou 14) selon la revendication 2, dans lequel la soupape unidirectionnelle (36) est une soupape à diaphragme.

4. Ventricule artificiel (12 ou 14) selon la revendication 1 ou 2, dans lequel la chambre (20 ou 30) a une forme ovoïde.

5. Ventricule artificiel (12 ou 14) selon la revendication 1 ou 2, comportant en outre :
une alimentation en énergie électrique (48) reliée électriquement au générateur de champ magnétique ;
un dispositif de commande (52) qui entraîne l'alimentation en énergie électrique (48) pour soit mettre sous tension soit mettre hors tension le générateur de champ magnétique ; et
un générateur de signal ECG (54) qui signale au dispositif de commande (52) lorsqu'il existe une diastole ventriculaire et une systole ventriculaire, dans lequel le dispositif de commande (52) entraîne l'alimentation en énergie électrique (48) pour mettre sous tension le générateur de champ magnétique lorsque le générateur de signal ECG (54) signale au dispositif de commande (52) qu'il existe une diastole ventriculaire et dans lequel le dispositif de commande (52) entraîne l'alimentation en énergie électrique (48) pour mettre hors tension le générateur de champ magnétique lorsque le générateur de signal ECG (54) signale au dispositif de commande (52) qu'il existe une systole ventriculaire.

6. Ventricule artificiel (12 ou 14) comprenant une entrée (16 ou 26) pour recevoir du sang ;
une sortie (18 ou 28) pour évacuer le sang ;
une chambre (20 ou 30) disposée entre l'entrée (16 ou 26) et la sortie (18 ou 28) ;
un mécanisme pour actionner le ventricule artificiel (12 ou 14) entre une configuration étendue et une configuration contractée, dans lequel, dans la configuration étendue, le sang s'écoule dans l'entrée (16 ou 26) et, dans la configuration contractée, le sang s'écoule hors de la sortie (18 ou 28) ; et
**caractérisé en ce que** la chambre (20 ou 30) a une paroi externe élastique (38) et le mécanisme pour actionner le ventricule artificiel (12 ou 14) entre la configuration étendue et la configuration contractée comporte :
un générateur de champ magnétique dans la paroi externe élastique (38) de la chambre (20 et 30) ; et
un matériau (46) dans la paroi externe élastique (38) de la chambre (20 ou 30) qui est attiré vers le générateur de champ magnétique lorsque le générateur de champ magnétique génère un champ magnétique, dans lequel le matériau (46) se déplace vers le générateur de champ magnétique lorsque le générateur de champ magnétique génère un champ magnétique et actionne ainsi le ventricule artificiel (12 ou 14) vers la configuration contractée en contractant la paroi externe élastique (38) de la chambre (20 ou 30), et dans lequel la paroi externe élastique (38) de la chambre (20 ou 30) actionne le ventricule artificiel (12 ou 14) vers la configuration étendue lorsque le générateur de champ magnétique ne génère pas de champ magnétique et que le matériau (46) n'est pas attiré vers le générateur de champ magnétique.

7. Ventricule artificiel (12 ou 14) selon la revendication 6, comportant en outre :
une alimentation en énergie électrique (48) reliée électriquement au générateur de champ magnétique ;
un dispositif de commande (52) qui entraîne l'alimentation en énergie électrique (48) pour soit mettre sous tension soit mettre hors tension le générateur de champ magnétique ; et
un générateur de signal ECG (54) qui signale au dispositif de commande (52) lorsqu'il existe une diastole ventriculaire et une systole ventriculaire, dans lequel le dispositif de commande (52) entraîne l'alimentation en énergie électrique (48) pour mettre sous tension le générateur de champ magnétique lorsque le générateur de signal ECG (54) signale au dispositif de commande (52) qu'il existe une diastole ventriculaire et dans lequel le dispositif de commande (52) entraîne l'alimentation en énergie électrique (48) pour mettre hors tension le générateur de champ magnétique lorsque le générateur de signal ECG (54) signale au dispositif de commande (52) qu'il existe une systole ventriculaire.
